# EUROPEAN PATENT SPECIFICATION

(11) **EP 4 070 785 B1**
(45) Date of publication and mention of the grant of the patent: **08.11.2023**
(21) Application number: 21166992.4
(22) Date of filing: 06.04.2021
(51) Int. Cl.: A61K 9/00, A61K 9/19, A61K 47/26, A61K 31/5377

(54) **LYOPHILIZED COMPOSITION OF COPANLISIB SALT**
LYOPHILISIERTE ZUSAMMENSETZUNG VON EINEM COPANLISIB-SALZ
COMPOSITION LYOPHILISÉE D'UN SEL DE COPANLISIB

(43) Date of publication of application: 12.10.2022
(73) Proprietor: Synthon B.V., 6545 CM Nijmegen (NL)
(72) Inventor: ZALUDEK, Borek, 67801 Blansko (CZ); LOBL, Jiri, 67801 Blansko (CZ)
(74) Representative: Dobsík, Martin

(56) References cited:
- WO-A1-2019/048527
- SCHWEMANN J J ET AL: "PRACTICAL FORMULATION AND PROCESS DEVELOPMENT OF FREEZE-DRIED PRODUCTS", PHARMACEUTICAL DEVELOPMENT AND TECHNOLOGY, NEW YORK, NY, US, vol. 10, no. 2, 1 January 2005 (2005-01-01), pages 151-173, XP008048993, ISSN: 1083-7450
- COSTANTINO ET AL: "Excipients for use in Lyophilized Pharmaceutical Peptide, Protein, and other Bioproducts", 1 January 2005 (2005-01-01), LYOPHILIZATION OF BIOPHARMACEUTICALS,, PAGE(S) 139 - 228, XP008159820, ISBN: 978-0-9711767-6-8 * the whole document *

## Description

### BRIEF DESCRIPTION OF THE PRESENT INVENTION

The presented invention relates to a lyophilized pharmaceutical formulation comprising:
a. Copanlisib salt;
b. A bulking agent selected from lactose or trehalose;
   and to a process for preparation thereof.

### DETAILED DESCRIPTION OF THE PRESENT INVENTION

The presented invention relates to a lyophilized pharmaceutical formulation comprising:
a. Copanlisib salt;
b. A bulking agent selected from lactose or trehalose;
   and to a process for preparation thereof.

The copanlisib salt is preferably hydrochloride salt or dihydrochloride salt or hydrogen phosphate salt or malate salt.
The formulation optionally comprises glycine. The ratio between the bulking agent selected from trehalose or lactose and glycine can be between 2: 5 to 5: 2 (parts by weight), said ratios of excipients being calculated on the anhydrous basis and the sum of the parts by weight of the sugar component and glycine is 7.

Glycine as Tg'-modifer (glass transition temperature modifier) capable of modifying a sufficiently Tg' of the pharmaceutical solution to higher temperatures and therefore improves the economy of process for preparation of the formulation.

The weight ratio between the copanlisib salt and the bulking agent can be between 1:2 and 1:2.5, preferably between 1:2.1 and 1:2.3. In the case both the bulking agent and glycine are used, the weight ratio between copanlisib or a salt thereof and sum of the bulking agent and glycine can be between 1:2 and 1:2.5, preferably between 1:2.2 and 1:2.4.

The bulking agent selected from lactose or trehalose can be used either in an anhydrous form or in a hydrated form. Trehalose is preferably used as trehalose dihydrate, lactose is preferably used as lactose monohydrate.

It has been surprisingly found that the use of lactose or trehalose provides better stability than other bulking agents disclosed in the prior art. Comparative trials have been performed against mannitol and glycine among others bulking agents, the results are disclosed in Examples 1 to 8.

In comparison with the formulation disclosed in WO2019/048527, the formulation of the presented invention has following advantages:
1. Better stability of the formulation;
2. Mannitol as a bulking agent can be risky due to the danger of vial breaking. There is no such risk when trehalose or lactose is used;
3. If mannitol crystallizes after lyophilization, it releases the associated water back into the already dried material, which accelerates the destabilization of the water sensitive drug. There is no such risk associated with use of when trehalose or lactose;
4. The formulation of presented invention shows good stability and solubility at pH higher than 5.5. WO2019/048527 shows in the examples that a pH higher than 5 is not suitable for the preparation of a pharmaceutical (bulk) solution, in particular in view of the possible precipitation of the dissolved material.

The presented invention further relates to a pharmaceutical solution comprising:
1. Copanlisib salt;
2. A bulking agent selected from trehalose or lactose;
3. A solvent.

The pharmaceutical solution further optionally comprises glycine.

The concentration of copanlisib salt thereof in the solution is preferably 30 mg/ml (calculated on copanlisib as a base). The salt of copanlisib is preferably hydrochloride salt or dihydrochloride salt or hydrogen phosphate salt or malate salt. In a preferred embodiment of the invention the copanlisib salt is formed in situ during the manufacturing process by contacting copanlisib with an acid in the solvent.

The bulking agent selected from lactose or trehalose can be used either in an anhydrous form or in a hydrated form. Trehalose is preferably used as trehalose dihydrate, lactose is preferably used as lactose monohydrate.

The weight ratio between copanlisib salt and the bulking agent (calculated as anhydrous form) can be between 1:2 and 1:2.5, preferably between 1:2.1 and 1:2.3. The concentration of trehalose (calculated as anhydrous form) in the pharmaceutical solution can be between 62.5 mg/ml and 72.5 mg/ml, preferably it is 67.5 mg/ml. The concentration of lactose (calculated as anhydrous form) in the pharmaceutical solution can be between 58.5 mg/ml and 68.5 mg/ml, preferably it is 63.2 mg/ml.

In the case glycine is used the ratio between the bulking agent and glycine can be between 2: 5 to 5: 2 (parts by weight), said ratios of excipients being calculated on the anhydrous basis and the sum of the parts by weight of the sugar component and glycine is 7 and the weight ratio between copanlisib or a salt thereof and the sum of the bulking agent and glycine can be between 1:2 and 1:2.5, preferably between 1:2.2 and 1:2.4. The concentration of trehalose (calculated as anhydrous form) or lactose (calculated as anhydrous form) in the pharmaceutical solution can be between 20 mg/ml and 70 mg/ml. The concentration of glycine in the pharmaceutical solution can be between 30 mg/ml and 60 mg/ml, preferably it is between 40 mg/ml and 50 mg/ml.

The solvent is preferably water, more preferably water for injection.

pH of the solution can be adjusted for example 2 M aqueous sodium hydroxide solution. Preferably, pH of the solution is adjusted to pH greater than 5.5, preferably to pH between 5.5 and 5.7.

The pharmaceutical solution can be further processed to a lyophilized dosage forms suitable for parenteral administration after reconstitution.

It has been surprisingly found that the stability of the prepared solutions remains unchanged for at least 72 hours, which is particularly advantageous for the industrial production of injectable dosage forms. The tested solutions remained clear and without precipitation for the indicated time. Also, the stability of the drug products prepared according to examples shows a significant improvement in terms of purity compared to the formulations disclosed in WO2019/048527 as is presented in Example 7.

The presented invention further relates to a process for preparation of lyophilized pharmaceutical formulation comprising:
a. Copanlisib salt;
b. A bulking agent selected form lactose or trehalose;
   and optionally glycine, the process comprising:
   1. Mixing the copanlisib salt with the bulking agent in a suitable solvent;
   2. Stiring the mixture to obtain a solution;
   3. Adjusting pH of the solution;
   4. Lyophilization of the solution.

The process steps are preferably performed under a protective atmosphere, for example using nitrogen or argon. The salt can be selected from copanlisib salt with hydrochloric acid or phosphoric acid (H₃PO₄) or malic acid. The suitable solvent is preferably water. pH of the solution is preferably adjusted to pH between 5.5 and 5.7. The formulation can optionally comprising glycine.

In a preferred embodiment of the invention the copanlisib salt is prepared "in situ" by contacting copanlisib base with an acid in the solvent. In the preferred embodiment of the invention the of lyophilized pharmaceutical formulation comprising:
a. Copanlisib salt;
b. A bulking agent selected form lactose or trehalose;
   is prepared by a process comprising:
   1. Mixing the copanlisib base with the bulking agent in a suitable solvent;
   2. Adding the acid;
   3. Stirring the mixture to obtain a solution;
   4. Adjustting pH of the solution;
   5. Lyophilization of the solution.

The pharmaceutical formulation can optionally comprising glycine. The process steps are preferably performed under a protective atmosphere, for example using nitrogen or argon. The concentration of copanlisib base in the solvent is preferably 30 mg/ml. The acid can be selected form hydrochloric acid or phosphoric acid (H₃PO₄) or malic acid. The acid can be added in a form of a solution of the acid in the suitable solvent. The molar ratio between copanlisib base and the hydrochloric acid can be between1:2 and 1:2.4. The molar ratio between copanlisib and phosphoric acid (H₃PO₄) or malic acid can be between 1:1 and 1:1.3. The mixture is then stirred for between 10 and 60 minutes to obtain a solution. For the preparation of said salts "in situ", it is advantageous to use the corresponding acids, i.e. hydrochloric acid or malic acid or phosphoric acid (H₃PO₄) in an excess of 10% with respect to their stoichiometric amount needed to form the salt. The benefit of this process is the surprising simplicity of the process and economic advantage, where the "in situ" preparation eliminates yield losses due to salt isolation by crystallization or precipitation, further material handling such as separation drying and subsequent characterization. It is generally known that each crystallization step can reduce the yield by 10 to 30% (w/w).

The suitable solvent is preferably water. pH of the solution is preferably adjusted to pH higher than 5.5, more preferably to pH between 5.5 and 5.7, advantageously with sodium hydroxide water solution (for example 2M solution).

The pharmaceutical solution for lyophilization preferably contains:
- 30 mg/ml of copanlisib (calculated as a copanlisib base) in the form of a soluble salt preferably selected from hydrochloride salt or dihydrochloride salt or hydrogen phosphate salt acid or malate salt. The salt can be added as a solid form or it can be prepared "in situ";
- Between 20 mg/ml and 70 mg/ml bulking agent (trehalose or lactose or hydrates thereof, preferably trehalose dihydrate or lactose monohydrate); and
- Optionally glycine in a concentration between 30 mg/ml and 60 mg/ml, preferably between 40 mg/ml and 50 mg/ml.

The concentration of the bulking agent in the pharmaceutical solution is preferably:
i. In the case of trehalose 67.5 mg/ml (calculated on trehalose anhydrous form);
ii. In the case of lactose 63.2 mg/ml (calculated on lactose anhydrous form).

In the case glycine is optionally used, the concentration of bulking agent trehalose or lactose is preferably between 20 mg/ml and 30 mg/ml (calculated on trehalose anhydrous form or lactose anhydrous form) and the ratio between the bulking agent and glycine can be between 2: 5 to 5: 2 (parts by weight), said ratios of excipients being calculated on the anhydrous basis and the sum of the parts by weight of the sugar component and glycine is 7.

The prepared pharmaceutical solution can be further processed to lyophilized injectable dosage forms as described in Examples or by a process comprising:
1. Sterile filtration of the pharmaceutical solution (through two consecutive filters with porosity of 0.2 micrometers;
2. Cleaning and depyrogenation of the vials; sterilization of the stoppers;
3. Filling and pre-closing of the vials;
4. Lyophilization and closing of the vials directly in the lyophilization chamber (under defined pressure, inert atmosphere of nitrogen can be also used);
5. Capping of the vials;
6. Visual inspection;
7. Labeling and packaging of the vials.

After reconstitution, for example in saline solution, the lyophilized formulation according to presented invention can be used for treatment conditions treatable by copanlisib or a salt thereof.

The invention will be further illustrated by the following examples.

### EXAMPLES

### Reference Example 1: Lyophilized formulation prepared according to WO2019/048527

Composition of the pharmaceutical solution:

| | |
|---|---|
| Copanlisib base | 40.0 mg/ml |
| Mannitol | 80.0 mg/ml, |
| Citric acid, anhydrous | 3.68 mg/ml |

2000 mg of Mannitol was mixed with 1152 mg of copanlisib dihydrochloride, 92 mg of citric acid anhydrous and 23 mL water for injection (WFI). The mixture was sonicated for 60 seconds. Pale yellow solution was formed. pH of the solution was then adjusted using 2M NaOH to pH 4.58. Volume was made up to 25 mL with WFI.

Solution was filtered using a 0.22-micron syringe filter (PVDF), filled into ISO 6R vials and semi-stoppered with two leg stoppers. Filling dose was 1.71 ml per vial, overfill corresponds with 8.4 mg of Copanlisib base.

In order to obtain the product without any risks of the cake collapse or any chemical degradation, the lyophilization process was conducted at the conservative parameters presented in the WO2019/048527.

Thus, filled vials were loaded in in the lyophilization chamber and lyophilized using Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 1:20 | -45 | Atm. |
| 3 | Freezing | 1:30 | -45 | Atm. |
| 4 | Freezing | 1:00 | -20 | Atm. |
| 5 | Annealing | 4:00 | -20 | Atm. |
| 6 | Freezing | 1:10 | -45 | Atm. |
| 7 | Freezing | 1:00 | -45 | Atm. |
| 8 | Main Drying | 0:10 | -45 | 0.100 |
| 9 | Main Drying | 1:00 | -5 | 0.100 |
| 10 | Main Drying | 20:00 | -5 | 0.100 |
| 11 | Secondary Drying | 1:50 | +35 | 0.100 |
| 12 | Secondary Drying | 6:00 | +35 | 0.100 |

In the end of the lyophilization process vials were closed under vacuum directly into the lyophilization chamber.

Prepared vials (after lyophilization) were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Stability study results (storage conditions 40 °C/ 75 % R.H.)

| **Sampling point** | **Start** | **1 month** | **2 months** | **3 months** |
|---|---|---|---|---|
| pH | 4.59 | 4.59 | 4.62 | 4.59 |
| Color | GY6-GY7 | GY6 | GY5-GY6 | GY6 |
| Clarity | < Ref. susp. I | < Ref. susp. I | < Ref. susp. I | < Ref. susp. I |
| Reconstitution time (seconds) | 22 | 21 | 29 | 26 |
| Copanlisib base (%) | 100.0 | 99.2 | 98.8 | 97.2 |
| Sum of impurities (% IN) | 1.98 | 2.36 | 2.06 | 2.79 |

### Example 2: Lyophilized formulation comprising Copanlisib dihydrochloride and trehalose

Composition of the pharmaceutical solution:

| | |
|---|---|
| Copanlisib base | 30.0 mg/ml |
| Trehalose | 67.5 mg/ml, |

3746.2 mg of trehalose dihydrate was mixed with 1728 mg of copanlisib dihydrochloride and 45 mL water for injection (WFI). The mixture was sonicated for 60 seconds. Pale yellow solution was formed.

pH of the solution was then adjusted using 2M NaOH to pH 5.57. Volume was made up to 50 mL with WFI.

Solution was filtered using a 0.22-micron syringe filter (PVDF), filled into ISO 6R vials and semi-stoppered with two leg stoppers. Filling dose was 2.28 ml per vial, overfill corresponds with 8.4 mg of Copanlisib base.

Filled vials were loaded in in the lyophilization chamber and lyophilized using Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 1:20 | -45 | Atm. |
| 3 | Freezing | 1:30 | -45 | Atm. |
| 4 | Freezing | 1:00 | -25 | Atm. |
| 5 | Annealing | 4:00 | -25 | Atm. |
| 6 | Freezing | 1:30 | -45 | Atm. |
| 7 | Freezing | 1:00 | -45 | Atm. |
| 8 | Main Drying | 0:10 | -45 | 0.100 |
| 9 | Main Drying | 1:00 | -20 | 0.100 |
| 10 | Main Drying | 31:00 | -20 | 0.100 |
| 11 | Secondary Drying | 4:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Stability study results (storage conditions 40 °C/ 75 % R.H.)

| **Sampling point** | **Start** | **1 month** | **2 months** | **3 months** |
|---|---|---|---|---|
| pH | 5.74 | 5.80 | 5.83 | 5.82 |
| Color | GY5 | GY5 | GY5 | GY5 |
| Clarity | < Ref. susp. I | < Ref. susp. I | < Ref. susp. I | < Ref. susp. I |
| Reconstitution time (seconds) | 29 | 33 | 27 | 35 |
| Copanlisib base (%) | 100.0 | 95.2 | 95.4 | 96.9 |
| Sum of impurities (% IN) | 1.76 | 1.82 | 2.01 | 1.98 |

### Example 3: Lyophilized formulation comprising Copanlisib dihydrochloride and lactose

Composition of the pharmaceutical solution:

| | |
|---|---|
| Copanlisib base | 30.0 mg/ml |
| Lactose | 63.2 mg/ml |

3507.6 mg of lactose monohydrate was mixed with 1728 mg of copanlisib dihydrochloride and 40 mL water for injection (WFI), sonicated for 4 minutes. Pale yellow solution was formed.

pH of the solution was then adjusted using 2M NaOH to pH 5.69. Volume was made up to 50 mL with WFI.

Solution was filtered using a 0.22-micron syringe filter (PVDF), filled into ISO 6R vials and semi-stoppered with two leg stoppers. Filling dose was 2.28 ml per vial, overfill corresponds with 8.4 mg of Copanlisib base.

Filled vials were loaded in in the lyophilization chamber and lyophilized using Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 1:20 | -45 | Atm. |
| 3 | Freezing | 1:30 | -45 | Atm. |
| 4 | Freezing | 1:00 | -18 | Atm. |
| 5 | Annealing | 4:00 | -18 | Atm. |
| 6 | Freezing | 1:30 | -45 | Atm. |
| 7 | Freezing | 1:00 | -45 | Atm. |
| 8 | Main Drying | 0:10 | -45 | 0.100 |
| 9 | Main Drying | 1:00 | -20 | 0.100 |
| 10 | Main Drying | 31:00 | -20 | 0.100 |
| 11 | Secondary Drying | 4:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

Stability study results (storage conditions 40 °C/ 75 % R.H.)

| **Sampling point** | **Start** | **1 month** | **2 months** | **3 months** |
|---|---|---|---|---|
| pH | 5,80 | 5,87 | 5,81 | 5,85 |
| Color | GY5-GY6 | GY5 | GY5 | GY5 |
| Clarity | < Ref. susp. I | < Ref. susp. I | < Ref. susp. I | < Ref. susp. I |
| Reconstitution time (seconds) | 42 | 34 | 29 | 35 |
| Copanlisib base (%) | 100.0 | 95.2 | 95.4 | 96.9 |
| Sum of impurities (% IN) | 1.76 | 1.87 | 2.02 | 1.86 |

### Example 4A, 4B, 4C: Lyophilized formulation comprising copanlisib dihydrochloride, trehalose or lactose, glycine

Composition of pharmaceutical solutions:

| | **Example 4A** | **Example 4B** | **Example 4C** |
|---|---|---|---|
| Copanlisib base | 30 mg/ml | 30 mg/ml | 30 mg/ml |
| Glycine | 70 mg/ml | 50 mg/ml | 50 mg/ ml |
| Trehalose | - | 20 mg /ml | - |
| Lactose | - | - | 20 mg/ml |

**Example 4A:** 1728 mg of copanlisib dihydrochloride were mixed with 3500 mg of glycine and 30 ml of water for injection (WFI) previously bubbled with nitrogen for 20 minutes. The mixture was stirred for 15 min at 400 rpm until dissolution. Yellow solution was formed.

pH of the solution was then adjusted using 2M NaOH to pH 5.60. Volume was made up to 50 mL with WFI. pH of the solution was 5.45, therefore pH was readjusted by 2 M NaOH solution to pH 5.60.

Solution was filtered using a 0.22-micron syringe filter (PVDF), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 4A | Start | 5°C/72h |
|---|---|---|
| Sum of impurities (% IN) | 1.24 | 1.16 |

**Example 4B:** 1728 mg of copanlisib dihydrochloride was mixed with 2500 mg of glycine, 1110 mg of trehalose dihydrate and 30 ml of water for injection (WFI) previously bubbled with nitrogen for 20 minutes. The mixture was stirred at 400 rpm for 15 minutes until dissolution. Yellow solution was formed.

pH of the solution was then adjusted using 2M NaOH to pH 5.61. Volume was made up to 50 mL with WFI. pH was readjusted by 2 M NaOH solution to 5.60.

Solution was filtered using a 0.22-micron syringe filter (PVDF), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 4B | Start | 5°C/72h |
|---|---|---|
| Sum of impurities (% IN) | 1.24 | 1.22 |

**Example 4C:** 1728 mg of copanlisib dihydrochloride was mixed with 2500 mg of glycine, 1050 mg of lactose monohydrate and 30 ml of water for injection (WFI) previously bubbled with nitrogen for 20 minutes. The mixture was stirred at 400 rpm for 15 minutes until dissolution. Yellow solution was formed.

pH of the solution was then adjusted using 2M NaOH to pH 5.60. Volume was made up to 50 mL with WFI. pH was readjusted by 2 M NaOH solution to 5.57.

Solution was filtered using a 0.22-micron syringe filter (PVDF), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 4C | Start | 5°C/72h |
|---|---|---|
| Sum of impurities (% IN) | 1.24 | 1.21 |

Vials prepared according to Examples 4A, 4B and 4C were loaded in in the lyophilization chamber and lyophilized using Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 2:00 | -45 | Atm. |
| 3 | Freezing | 1:30 | -45 | Atm. |
| 4 | Freezing | 1:00 | -10 | Atm. |
| 5 | Annealing | 4:00 | -10 | Atm. |
| 6 | Freezing | 1:10 | -45 | Atm. |
| 7 | Freezing | 1:30 | -45 | Atm. |
| 8 | Main Drying | 0:10 | -45 | 0.100 |
| 9 | Main Drying | 4:00 | -5 | 0.100 |
| 10 | Main Drying | 20:30 | -5 | 0.100 |
| 11 | Secondary Drying | 5:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Example 4A Glycine: Stability study results (storage conditions 40 °C/ 75 % R.H.)

| **Sampling point** | **Start** | **1 month** | **2 months** | **3 months** |
|---|---|---|---|---|
| pH | 5.58 | 5.66 | 5.62 | 5.53 |
| Color | GY5-GY6 | GY5 | GY5 | GY5 |
| Clarity | < Ref. susp. I | Ref. susp. III | Ref. susp. III | Ref. susp. III |
| Reconstitution time (seconds) | 45 | 42 | 65 | 60 |
| Copanlisib base (%) | 100.0 | 97.0 | 94.8 | 95.0 |
| Sum of impurities (% IN) | 1.25 | 2.47 | 3.14 | 2.44 |

### Example 4B Trehalose+Glycine: Stability study results (storage conditions 40 °C/ 75 % R.H.)

| **Sampling point** | **Start** | **1month** | **2 months** | **3 months** |
|---|---|---|---|---|
| pH | 5.56 | 5.59 | 5.62 | 5.53 |
| Color | GY5-GY6 | GY6 | GY5-GY6 | GY5-GY6 |
| Clarity | < Ref. susp. I | < Ref. susp. I | < Ref. susp. I | < Ref. susp. I |
| Reconstitution time (seconds) | 53 | 62 | 22 | 15 |
| Copanlisib base (%) | 100.0 | 100.1 | 95.8 | 95.5 |
| Sum of impurities (% IN) | 1.24 | 1.48 | 1.36 | 1.42 |

### Example 4C Lactose+Glycine: Stability study results (storage conditions 40 °C/ 75 % R.H.)

| **Sampling point** | **Start** | **1 month** | **2 months** | **3 months** |
|---|---|---|---|---|
| pH | 5.54 | 5.57 | 5.60 | 5.54 |
| Color | GY5-GY6 | GY5-GY6 | GY5 | GY5 |
| Clarity | < Ref. susp. I | < Ref. susp. I | < Ref. susp. I | Ref. susp. II |
| Reconstitution time (seconds) | 52.00 | 22.00 | 18.00 | 28.00 |
| Copanlisib base (%) | 100.0 | 98.9 | 96.5 | 97.7 |
| Sum of impurities (% IN) | 1.40 | 1.51 | 1.46 | 1.40 |

### Examples 5A, 5B, 5C: Lyophilized formulation comprising copanlisib hydrogenphosphate, trehalose or lactose, glycine

Composition of pharmaceutical solutions:

| | **Example 5A** | **Example 5B** | **Example 5C** |
|---|---|---|---|
| Copanlisib base | 30 mg/ml | 30 mg/ml | 30 mg/ml |
| Glycine | 70 mg/ml | 50 mg/ml | 50 mg/ ml |
| Trehalose | - | 20 mg /ml | - |
| Lactose | - | - | 20 mg/ml |

**Example 5A:** 1500 mg of copanlisib base was mixed with 3500 mg of Glycine and 20 mL of water for injection (WFI). 5 ml Phosphoric acid (H₃PO₄) in form of diluted solution (88 mg/ml in water) was added and the mixture was stirred for 15 minutes at 400 rpm until dissolution. Yellow solution was formed.

pH of the solution was then adjusted using 2M NaOH to pH 5.63. Volume was made up to 50 mL with WFI, pH was not changed.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 5A | Start | 5°C/72h |
|---|---|---|
| Sum of impurities (% IN) | 1.12 | 1.04 |

**Example 5B:** 1500 mg of copanlisib base was mixed with 2500 mg of Glycine, 1110 mg of trehalose dihydrate and 20 mL of water for injection (WFI). 5 ml of Phosphoric acid in form of diluted solution (88 mg/ml in water) was added. The mixture was stirred for 15 minutes at 400 rpm until dissolution. Yellow solution was formed.
pH of the solution was then adjusted using 2M NaOH to pH 5.67. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 5B | Start | 5°C/72h |
|---|---|---|
| Sum of impurities (% IN) | 1.12 | 1.07 |

**Example 5C:** 1500 mg of Copanlisib base was mixed with 2500 mg of Glycine, 1050 mg of Lactose monohydrate and 20 mL of water for injection (WFI). 5 ml of Phosphoric acid in form of diluted solution (88 mg/ml in water) was added and the mixture stirred for 15 minutes at 400 rpm until dissolution. Yellow solution was formed.

pH of the solution was then adjusted using 2M NaOH to pH 5.63. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 5C | Start | 5°C/72h |
|---|---|---|
| Sum of impurities (% IN) | 1.12 | 1.10 |

Vials prepared according to Examples 5A, 5B and 5C were loaded in the lyophilization chamber and lyophilized using Christ Epsilon 2-6D according to following program:

| No. | Phase | Time (hh:mm) | Shelf (temperature °C) | Vacuum (mbar) |
|---|---|---|---|---|
| 1 | Precooling/Loading | | +20 | Atm. |
| 2 | Freezing | 2:00 | -45 | Atm. |
| 3 | Freezing | 1:30 | -45 | Atm. |
| 4 | Freezing | 1:00 | -10 | Atm. |
| 5 | Annealing | 4:00 | -10 | Atm. |
| 6 | Freezing | 1:10 | -45 | Atm. |
| 7 | Freezing | 2:30 | -45 | Atm. |
| 8 | Main Drying | 0:10 | -45 | 0.100 |
| 9 | Main Drying | 4:00 | -5 | 0.100 |
| 10 | Main Drying | 20:30 | -5 | 0.100 |
| 11 | Secondary Drying | 5:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Example 5A Glycine: Stability study results (storage conditions 40 °C/ 75 % R.H.)

| **Sampling point** | **Start** | **1 month** | **2 months** | **3 months** |
|---|---|---|---|---|
| pH | 5.80 | 5.72 | 5.72 | 5.79 |
| Color | GY6-GY7 | GY6 | GY6 | GY5-GY6 |
| Clarity | < Ref. susp. I | < Ref. susp. I | Ref. susp. III | Ref. susp. III |
| Reconstitution time (seconds) | 28 | 22 | 20 | 45 |
| Copanlisib base (%) | 100.0 | 100.3 | 100.6 | 99.1 |
| Sum of impurities (% IN) | 1.03 | 1.87 | 2.66 | 3.34 |

### Examples 6A, 6B, 6C: Lyophilized formulation comprising copanlisib malate, trehalose or lactose, glycine

Composition of pharmaceutical solutions:

| | **Example 6A** | **Example 6B** | **Example 6C** |
|---|---|---|---|
| Copanlisib base | 30 mg/ml | 30 mg/ml | 30 mg/ml |
| Glycine | 70 mg/ml | 50 mg/ml | 50 mg/ ml |
| Trehalose | - | 20 mg /ml | - |
| Lactose | - | - | 20 mg/ml |

**Example 6A:** 1500 mg of Copanlisib base was mixed with 3500 mg of Glycine and 20 mL of water for injection (WFI). 5ml of Malic acid in form of diluted solution (97 mg/ml in water) was added and the mixture stirred for 15 minutes at 400 rpm until dissolution. Yellow solution was formed.

pH of the solution was then adjusted using 2M NaOH to pH 5.63. Volume was made up to 50 mL with WFI. pH was readjusted by 2 M NaOH solution to 5.61.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 6A | Start | 5°C/72h |
|---|---|---|
| Sum of impurities (% IN) | 1.12 | 1.15 |

**Example 6B:** 1500 mg of copanlisib base was mixed with 2500 mg of Glycine, 1110 mg of Trehalose dihydrate and 20 mL of water for injection (WFI). 5 ml of Malic acid in form of diluted solution (97 mg/ml in water) was added and the mixture was stirred for 15 minutes at 400 rpm until dissolution. Pale yellow solution was formed.

pH of the solution was then adjusted using 2M NaOH to pH 5.64. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 6B | Start | 5°C/72h |
|---|---|---|
| Sum of impurities (% IN) | 1.09 | 1.03 |

**Example 6C:** 1500 mg of Copanlisib base was mixed with 2500 mg of Glycine, 1050 mg of Lactose monohydrate and 20 mL of water for injection (WFI). 5 ml of Malic acid in form of diluted solution (97 mg/ml in water) was added and the mixture stirred for 15 minutes at 400 rpm until dissolution. Pale yellow solution was formed.

pH of the solution was then adjusted using 2M NaOH to pH 5.67. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 6C | Start | 5°C/72h |
|---|---|---|
| Sum of impurities (% IN) | 1.12 | 1.04 |

Vials prepared according to Examples 6A, 6B and 6C were loaded in in the lyophilization chamber and lyophilized using Christ Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 2:00 | -45 | Atm. |
| 3 | Freezing | 1:30 | -45 | Atm. |
| 4 | Freezing | 1:00 | -10 | Atm. |
| 5 | Annealing | 4:00 | -10 | Atm. |
| 6 | Freezing | 1:10 | -45 | Atm. |
| 7 | Freezing | 2:30 | -45 | Atm. |
| 8 | Main Drying | 0:10 | -45 | 0.100 |
| 9 | Main Drying | 4:00 | -5 | 0.100 |
| 10 | Main Drying | 20:30 | -5 | 0.100 |
| 11 | Secondary Drying | 5:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Example 6A Glycine: Stability study results (storage conditions 40 °C/ 75 % R.H.)

| **Sampling point** | **Start** | **1 month** | **2 months** | **3 months** |
|---|---|---|---|---|
| pH | 5.60 | 5.55 | 5.58 | 5,59 |
| Color | GY6-GY7 | GY6-GY7 | GY5 | GY5 |
| Clarity | < Ref. susp. I | Ref. susp. II-III | Ref. susp. III | Ref. susp. III |
| Reconstitution time (seconds) | 38 | >10min) | >10min | >10min |
| Copanlisib base (%) | 100.0 | 102.4 | 100.1 | 98.5 |
| Sum of impurities (% IN) | 0.95 | 1.32 | 1.79 | 2.30 |

### Example 6B Trehalose+Glycine: Stability study results (storage conditions 40 °C/ 75 % R.H.)

| **Sampling point** | **Start** | **1month** | **2 months** | **3 months** |
|---|---|---|---|---|
| pH | 5.57 | 5.52 | 5.53 | 5.53 |
| Color | GY6-GY7 | GY6-GY7 | GY5-GY6 | GY5 |
| Clarity | < Ref. susp. I | < Ref. susp. I | < Ref. susp. I | < Ref. susp. I |
| Reconstitution time (seconds) | 26 | 15 | 24 | 18 |
| Copanlisib base (%) | 100.0 | 102.0 | 99.8 | 100.0 |
| Sum of impurities (% IN) | 0.89 | 1.12 | 1.22 | 1.30 |

### Example 6C Lactose+Glycine: Stability study results (storage conditions 40 °C/ 75 % R.H.)

| **Sampling point** | **Start** | **1 month** | **2 months** | **3 months** |
|---|---|---|---|---|
| pH | 5.61 | 5.56 | 5.56 | 5.56 |
| Color | GY6 | GY6-GY7 | GY5-GY6 | GY5 |
| Clarity | < Ref. susp. I | < Ref. susp. I | < Ref. susp. I | < Ref. susp. I |
| Reconstitution time (seconds) | 28 | 15 | 32 | 18 |
| Copanlisib base (%) | 100.0 | 101.9 | 99.7 | 99.7 |
| Sum of impurities (% IN) | 0.92 | 1.14 | 1.23 | 1.14 |

### Examples 7A, 7B: Lyophilized formulation comprising copanlisib hydrogenphosphate, trehalose or lactose, glycine

Composition of pharmaceutical solutions:

| | **Example 7A** | **Example 7B** |
|---|---|---|
| Copanlisib base | 30 mg/ml | 30 mg/ml |
| Glycine | 40 mg/ml | 40 mg/ ml |
| Trehalose | 30 mg /ml | - |
| Lactose | - | 30 mg/ml |

Example 7A: 1500 mg of copanlisib base was mixed with 2000 mg of Glycine, 1665 mg of Trehalose dihydrate and 20 mL of water for injection (WFI). 5 ml of Phosphoric acid (H3PO4) in form of diluted solution (88 mg/ml in water) was added and the mixture was stirred for 15 minutes at 400 rpm until dissolution. Yellow solution was formed.

pH of the solution was then adjusted using 2M NaOH to pH 5.64. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 7A | Start | 5°C/72h |
|---|---|---|
| Sum of impurities (% IN) | 1.09 | 1.06 |

**Example 7B:** 1500 mg of copanlisib base was mixed with 2000 mg of Glycine, 1575 mg of Lactose and 20 mL of water for injection (WFI). 5 ml of Phosphoric acid in form of diluted solution (88 mg/ml in water) was added and the mixture was stirred for 15 minutes at 400 rpm until dissolution. Yellow solution was formed.
pH of the solution was then adjusted using 2M NaOH to pH 5.66. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 7B | Start | 5°C/72h |
|---|---|---|
| Sum of impurities (% IN) | 1.09 | 1.07 |

Vials prepared according to 7A and 7B were loaded in in the lyophilization chamber and lyophilized using Christ Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 2:00 | -45 | Atm. |
| 3 | Freezing | 1:30 | -45 | Atm. |
| 4 | Freezing | 1:00 | -10 | Atm. |
| 5 | Annealing | 4:00 | -10 | Atm. |
| 6 | Freezing | 1:10 | -45 | Atm. |
| 7 | Freezing | 2:30 | -45 | Atm. |
| 8 | Main Drying | 0:10 | -45 | 0.100 |
| 9 | Main Drying | 4:00 | -5 | 0.100 |
| 10 | Main Drying | 20:30 | -5 | 0.100 |
| 11 | Secondary Drying | 5:00 | +35 | 0.100 |
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Example 7A Trehalose+Glycine: Stability study results (storage conditions 40 °C/ 75 % R.H.)

| **Sampling point** | **Start** | **1 month** | **2 months** | **3 months** |
|---|---|---|---|---|
| pH | 5.79 | 5.75 | 5.70 | 5.75 |
| Color | GY6-GY7 | GY6-GY7 | GY6 | GY6 |
| Clarity | < Ref. susp. I | < Ref. susp. I | < Ref. susp. I | < Ref. susp. I |
| Reconstitution time (seconds) | 20 | 20 | 20 | 15 |
| Copanlisib base (%) | 100.0 | 101.3 | 101.3 | 97.6 |
| Sum of impurities (% IN) | 1.09 | 1.16 | 1.22 | 1.32 |

### Example 7B Lactose+Glycine: Stability study results (storage conditions 40 °C/ 75 % R.H.)

| **Sampling point** | **Start** | **1 month** | **2 months** | **3 months** |
|---|---|---|---|---|
| pH | 5.72 | 5.70 | 5.67 | 5.74 |
| Color | GY6-GY7 | GY6-GY7 | GY5-GY6 | GY6 |
| Clarity | < Ref. susp. I | < Ref. susp. I | < Ref. susp. I | < Ref. susp. I |
| Reconstitution time (seconds) | 20 | 20 | 20 | 20 |
| Copanlisib base (%) | 100.0 | 100.7 | 102.0 | 97.9 |
| Sum of impurities (% IN) | 1.04 | 1.23 | 1.28 | 1.38 |

### Example 8A, 8B: Lyophilized formulation comprising copanlisib malate, trehalose or lactose, glycine

Composition of pharmaceutical solutions:

| | **Example 8A** | **Example 8B** |
|---|---|---|
| Copanlisib base | 30 mg/ml | 30 mg/ml |
| Glycine | 40 mg/ml | 40 mg/ ml |
| Trehalose | 30 mg /ml | - |
| Lactose | - | 30 mg/ml |

**Example 8A:** 1500 mg of copanlisib base was mixed with 2000 mg of Glycine and 1665 mg of trehalose dihydrate and 20 mL of water for injection (WFI). 5 ml of Malic acid in form of diluted solution (97 mg/ml in water) was added and the mixture was stirred for 15 minutes at 400 rpm until dissolution. Pale yellow solution was formed.

pH of the solution was then adjusted using 2M NaOH to pH 5.68. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 8A | Start | 5°C/72h |
|---|---|---|
| Sum of impurities (% IN) | 1.09 | 1.05 |

**Example 8B:** 1500 mg of Copanlisib base was mixed with 2000 mg of Glycine, 1575 mg of Lactose monohydrate and 20 mL of water for injection (WFI). 5 ml of Malic acid in form of diluted solution (97 mg/ml in water) was added and the mixture was stirred for 15 minutes at 400 rpm until dissolution. Pale yellow solution was formed.

pH of the solution was then adjusted using 2M NaOH to pH 5.71. Volume was made up to 50 mL with WFI.

Solution was filtered using a 0.22-micron syringe filter (PES), filled into ISO 6R vials and semi-stoppered with two leg stoppers. Filling dose was 2.28 ml per vial, overfill corresponds with 8.4 mg of Copanlisib base.

The pharmaceutical solution was analyzed after filtration and after 72 hours of storages at 5°C. Purity was determined by HPLC (Waters Alliance) with UV detection. No precipitation was seen in the pharmaceutical solution.

| Pharmaceutical solution 8B | Start | 5°C/72h |
|---|---|---|
| Sum of impurities (% IN) | 1.09 | 1.10 |

Vials prepared according to 8A and 8B were loaded in in the lyophilization chamber and lyophilized using Christ Epsilon 2-6D according to following program:

| **No.** | **Phase** | **Time (hh:mm)** | **Shelf (temperature °C)** | **Vacuum (mbar)** |
|---|---|---|---|---|
| 1 | Precooling/Loading | | +20 | Atm. |
| 2 | Freezing | 2:00 | -45 | Atm. |
| 3 | Freezing | 1:30 | -45 | Atm. |
| 4 | Freezing | 1:00 | -10 | Atm. |
| 5 | Annealing | 4:00 | -10 | Atm. |
| 6 | Freezing | 1:10 | -45 | Atm. |
| 7 | Freezing | 2:30 | -45 | Atm. |
| 8 | Main Drying | 0:10 | -45 | 0.100 |
| 9 | Main Drying | 4:00 | -5 | 0.100 |
| 10 | Main Drying | 20:30 | -5 | 0.100 |
| 11 | Secondary Drying | 5:00 | +35 | 0.100 |

| **No.** | **Phase** | **Time (hh:mm)** | **Shelf (temperature °C)** | **Vacuum (mbar)** |
|---|---|---|---|---|
| 12 | Secondary Drying | 6:00 | +35 | 0.100 |

In the end of the lyophilization process vials were closed under vacuum directly into the lyophilization chamber.

Prepared vials were put on stability study at 40 °C / 75 % R.H. Stability study results are presented in following table. Purity was determined by HPLC (Waters Alliance) with UV detection. Physical parameters were evaluated using method according to Ph. Eur. (pH, color, clarity).

After reconstitution with 4.4 ml of saline (0.9% NaCl), concentration of formed solution is 15 mg/ml of Copanlisib base.

### Example 8A Trehalose+Glycine: Stability study results (storage conditions 40 °C/ 75 % R.H.)

| **Sampling point** | **Start** | **1month** | **2 months** | **3 months** |
|---|---|---|---|---|
| pH | 5.64 | 5.64 | 5.59 | 5.64 |
| Color | GY7 | GY6 | GY6 | GY6 |
| Clarity | < Ref. susp. I | < Ref. susp. I | < Ref. susp. I | < Ref. susp. I |
| Reconstitution time (seconds) | 25 | 19 | 28 | 18 |
| Copanlisib base (%) | 100.0 | 98.9 | 98.7 | 99.1 |
| Sum of impurities (% IN) | 1.00 | 1.08 | 1.20 | 1.21 |

### Example 8B Lactose+Glycine: Stability study results (storage conditions 40 °C/ 75 % R.H.)

| **Sampling point** | **Start** | **1month** | **2 months** | **3 months** |
|---|---|---|---|---|
| pH | 5.61 | 5.62 | 5.55 | 5.66 |
| Color | GY7 | GY6 | GY5-GY6 | GY5-GY6 |
| Clarity | < Ref. susp. I | < Ref. susp. I | < Ref. susp. I | < Ref. susp. I |
| Reconstitution time (seconds) | 23 | 22 | 25 | 18 |
| Copanlisib base (%) | 100.0 | 99.1 | 100.1 | 99.2 |
| Sum of impurities (% IN) | 1.01 | 1.16 | 1.22 | 1.29 |

### Example 7:

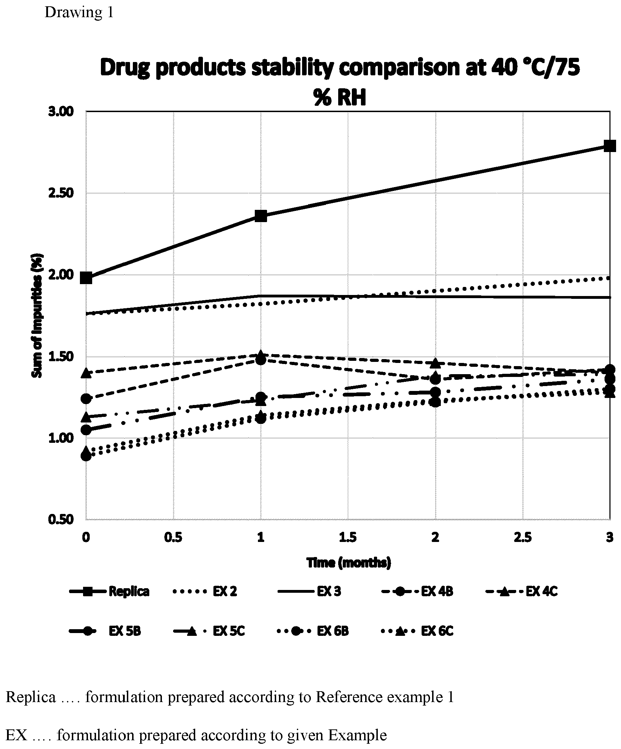

In drawing 1 the stability data of lyophilized formulations comprising copanlisib or a salt thereof and a bulking agent selected from trehalose or lactose and optionally glycine are depicted.

It can be concluded that stability (expressed as the change of sum of impurities in time) of lyophilized formulation according to presented invention (EX 2, EX 3, EX 4B, EX 4C, EX 5B, EX 5C, EX 6B, EX 6C) is significantly better that the stability of lyophilized formulation prepared according to prior art WO2019/048527 (Example 1, Replica in the chart).

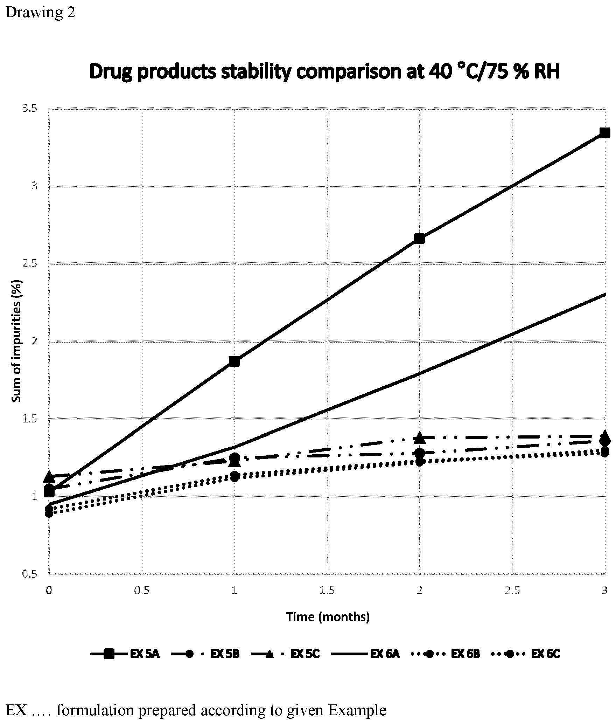

In drawing 2 stabilities of formulations comprising only glycine and combination of glycine and trehalose or lactose are depicted.

It can be concluded that stabilities of formulation comprising glycine only (EX 5A, EX 6A) are much worse that stabilities of formulation comprising glycine and trehalose or lactose (EX 5B, EX 5C, EX 6B, EX 6C).

## Claims

1. A lyophilized pharmaceutical formulation comprising:
a. copanlisib salt
b. a bulking agent selected from lactose or trehalose

2. The formulation according to claim 1 wherein the salt of copanlisib is hydrochloride salt or dihydrochloride salt or hydrogen phosphate salt or malate salt.

3. The formulation according to claims 1 or 2 further comprising glycine.

4. The formulation according to claim 3 wherein the ratio between the bulking agent selected from lactose or trehalose and glycine is between 2:5 and 5:2 (parts by weight), said ratios of excipients being calculated on the anhydrous basis and the sum of the parts by weight of the bulking agent and glycine is 7.

5. A process for preparation of the formulation according to claims 1 or 2 comprising:
a. mixing the copanlisib salt with the bulking agent in a suitable solvent
b. stirring the mixture to obtain a solution
c. adjusting pH of the solution
d. lyophilization of the solution

6. A process for preparation of the formulation according to claims 1 or 2 comprising:
a. mixing the copanlisib base with the bulking agent in a suitable solvent
b. adding the acid
c. stirring the mixture to obtain a solution
d. adjusting pH of the solution
e. lyophilization of the solution.

7. The process according to claims 5 or 6 wherein the formulation further comprises glycine.

8. The process according to any one of claims 5 to 7 wherein the suitable solvent is water

9. The process according to any one of claims 5 to 8 wherein pH of the solution is adjusted to pH higher than 5.5.

10. The process according to claim 9 wherein pH of the solution is adjusted to pH between 5.5 and 5.7.

11. A pharmaceutical solution comprising
a. 30 mg/ml of copanlisib salt (calculated as copanlisib base)
b. between 20 mg/ml and 70 mg/ml of bulking agent selected from trehalose or lactose or hydrates thereof (calculated on trehalose or lactose anhydrous form)

12. The solution according to claim 11 wherein the copanlisib salt is hydrochloride salt or dihydrochloride salt or hydrogen phosphate salt or malate salt

13. The solution according to claims 11 or 12 further comprising glycine in a concentration between 30 mg/ml and 60 mg/ml.

14. The solution according to claim 13 wherein the ratio between the bulking agent and glycine is between 2: 5 to 5: 2 (parts by weight), said ratios of excipients being calculated on the anhydrous basis and the sum of the parts by weight of the sugar component and glycine is 7.

## Patentansprüche

1. Lyophilisierte pharmazeutische Formulierung, umfassend:
a. Copanlisib-Salz
b. einen Füllstoff, ausgewählt aus Laktose oder Trehalose.

2. Formulierung nach Anspruch 1, wobei das Salz von Copanlisib Hydrochlorid-Salz oder Dihydrochlorid-Salz oder Hydrogenphosphat-Salz oder Malat-Salz ist.

3. Formulierung nach Ansprüchen 1 oder 2, die des Weiteren Glycin umfasst.

4. Formulierung nach Anspruch 3, wobei das Verhältnis zwischen dem aus Lactose oder Trehalose ausgewählten Füllstoff und Glycin zwischen 2:5 und 5:2 (Teile bezogen auf das Gewicht) ist, wobei die Verhältnisse der Hilfsstoffe auf der wasserfreien Basis berechnet sind, und die Summe der Teile bezogen auf das Gewicht des Füllstoffs und des Glycins 7 beträgt.

5. Verfahren zur Herstellung der Formulierung gemäß Ansprüchen 1 oder 2, umfassend:
a. Mischen des Copanlisib-Salzes mit dem Füllstoff in einem geeigneten Lösungsmittel
b. Rühren der Mischung, um eine Lösung zu erhalten
c. Einstellen des pH der Lösung
d. Lyophilisierung der Lösung

6. Verfahren zur Herstellung der Formulierung gemäß Ansprüchen 1 oder 2, umfassend:
a. Mischen der Copanlisib-Base mit dem Füllstoff in einem geeigneten Lösungsmittel
b. Zugeben der Säure
c. Rühren der Mischung, um eine Lösung zu erhalten
d. Einstellen des pH der Lösung
e. Lyophilisierung der Lösung.

7. Verfahren nach Ansprüchen 5 oder 6, wobei die Formulierung des Weiteren Glycin umfasst.

8. Verfahren nach einem beliebigen der Ansprüche 5 bis 7, wobei das geeignete Lösungsmittel Wasser ist.

9. Verfahren nach einem beliebigen der Ansprüche 5 bis 8, wobei der pH der Lösung auf einen pH von höher als 5,5 eingestellt wird.

10. Verfahren nach Anspruch 9, wobei der pH der Lösung auf einen pH zwischen 5,5 und 5,7 eingestellt wird.

11. Pharmazeutische Lösung, umfassend
a. 30 mg/ml Copanlisib-Salz (berechnet als Copanlisib-Base)
b. zwischen 20 mg/ml und 70 mg/ml Füllstoff, ausgewählt aus Trehalose oder Lactose oder Hydraten davon (berechnet an wasserfreier Form von Trehalose oder Lactose)

12. Lösung nach Anspruch 11, wobei das Copanlisib-Salz Hydrochlorid-Salz oder Dihydrochlorid-Salz oder Hydrogenphosphat-Salz oder Malat-Salz ist.

13. Lösung nach Ansprüchen 11 oder 12, die des Weiteren Glycin in einer Konzentration zwischen 30 mg/ml und 60 mg/ml umfasst.

14. Lösung nach Anspruch 13, wobei das Verhältnis zwischen dem Füllstoff und Glycin zwischen 2:5 bis 5:2 (Teile bezogen auf das Gewicht) ist, wobei die Verhältnisse der Hilfsstoffe auf der wasserfreien Basis berechnet sind, und die Summe der Teile bezogen auf das Gewicht der Zuckerkomponente und des Glycins 7 ist.

## Revendications

1. Une formulation pharmaceutique lyophilisée comprenant :
a. un sel de copanlisib
b. un agent de charge choisi parmi le lactose ou le tréhalose

2. La formulation selon la revendication 1, dans laquelle le sel de copanlisib est un sel de chlorhydrate ou un sel de dichlorhydrate ou un sel de phosphate d'hydrogène ou du sel de malate.

3. La formulation selon les revendications 1 ou 2, comprenant en outre de la glycine.

4. La formulation selon la revendication 3, dans laquelle le rapport entre l'agent de charge choisi parmi le lactose ou le tréhalose et la glycine est compris entre 2/5 et 5/2 (parties en poids), lesdits rapports d'excipients étant calculés sur la base anhydre et la somme des parties en poids de l'agent de charge et de la glycine étant de 7.

5. Un procédé de préparation de la formulation selon les revendications 1 ou 2, comprenant :
a. le mélange du sel de copanlisib avec l'agent de charge dans un solvant approprié
b. l'agitation du mélange pour obtenir une solution
c. l'ajustement du pH de la solution
d. la lyophilisation de la solution

6. Un procédé de préparation de la formulation selon les revendications 1 ou 2, comprenant :
a. le mélange de la base de copanlisib avec l'agent de charge dans un solvant approprié
b. l'ajout de l'acide
c. l'agitation du mélange pour obtenir une solution
d. l'ajustement du pH de la solution
e. la lyophilisation de la solution.

7. Le procédé selon les revendications 5 ou 6, dans lequel la formulation comprend en outre de la glycine.

8. Le procédé selon l'une quelconque des revendications 5 à 7, dans lequel le solvant approprié est l'eau.

9. Le procédé selon l'une quelconque des revendications 5 à 8 dans lequel le pH de la solution est ajusté à un pH supérieur à 5,5.

10. Le procédé selon la revendication 9 dans lequel le pH de la solution est ajusté à un pH compris entre 5,5 et 5,7.

11. Une solution pharmaceutique comprenant :
a. 30 mg/ml de sel de copanlisib (calculé en tant que base de copanlisib)
b. de 20 mg/ml à 70 mg/ml d'agent de charge choisi parmi le tréhalose ou le lactose ou leurs hydrates (calculé en tant que forme anhydre du tréhalose ou du lactose)

12. La solution selon la revendication 11, dans laquelle le sel de copanlisib est du sel de chlorhydrate ou du sel de dichlorhydrate ou du sel de phosphate d'hydrogène ou du sel de malate.

13. La solution selon les revendications 11 ou 12, comprenant en outre de la glycine à une concentration comprise entre 30 mg/ml et 60 mg/ml

14. La solution selon la revendication 13, dans laquelle le rapport entre l'agent de charge et la glycine est compris entre 2/5 et 5/2 parties en poids), lesdits rapports d'excipients étant calculés sur la base anhydre et la somme des parties en poids du composant sucre et glycine étant de 7.
